# EUROPEAN PATENT APPLICATION

(11) **EP 3 651 163 A1**
(43) Date of publication of application: **13.05.2020**
(21) Application number: 18204589.8
(22) Date of filing: 06.11.2018
(51) Int. Cl.: G16H 50/30, G16H 50/20, G16H 40/20, A61B 5/00, G06T 7/00, A61B 6/02, A61B 5/055, G06N 3/08

(54) **MEDICAL IMAGE DEVICE AND OPERATING METHOD**

(71) Applicant: Koninklijke Philips N.V., 5656 AG Eindhoven (NL)
(72) Inventor: SAALBACH, Axel, 5656 AE Eindhoven (NL); BROSCH, Tom, 5656 AE Eindhoven (NL); HARDER, Tim Philipp, 5656 AE Eindhoven (NL); DESHPANDE, Hrishikesh Narayanrao, 5656 AE Eindhoven (NL); SCHWAB, Evan, 5656 AE Eindhoven (NL); BALTRUSCHAT, Ivo Matteo, 5656 AE Eindhoven (NL); WIEMKER, Rafael, 5656 AE Eindhoven (NL)
(74) Representative: de Haan, Poul Erik

(57) **Abstract**

This application proposes an improved medical imaging device enabling a timely communication of critical findings. The medical imaging device comprises an image acquisition unit, adapted to acquire image data of a subject to be imaged. The medical imaging device further comprises a local data processing device having an artificial-intelligence-module, AI-module, adapted to automatically detect a finding on basis of the acquired image data and to determine a priority status of the detected finding. Further, the medical imaging device comprises a notification module, adapted to provide, if the determined priority status reaches or exceeds a notification threshold, notification data containing the detected finding. The application further proposes a medical imaging system, a method of operating a medical imaging device, a computer program element and a computer-readable medium having stored the computer program element.

## Description

### FIELD OF THE INVENTION

The present invention relates to medical imaging. In particular, it relates to a medical imaging device, as well as a method of operating an medical imaging device, a computer program element, and a computer readable medium.

### BACKGROUND OF THE INVENTION

In medical imaging, timely communication of in particular a critical finding at examined subjects may be desired. There may even be cases where timely communication of such a finding is mandated by, for example, institutional or statutory requirements. By way of example, there may exist different categories of noticeable or actionable finding which may require recognition and/or communication within minutes, hours or days. Especially for the minute-based category, immediate action or at least immediate communication may be required in order to avoid deterioration or even mortality.

In addition, in medical imaging, a time elapsing between image acquisition and evaluation of the same by a radiologist, physician or the like may affect the overall time of treatment. Typically, radiologists, physicians etc. work through a reading list sequentially, or guided by a manual prioritization process. In particular for an unexpected finding, this process may cause unnecessary delays in capturing a finding.

US 2008/0091473 A1 describes a notification system for a medical imaging device, in which a notification is sent informing about the progress of the image processing or its completion.

### SUMMARY OF THE INVENTION

There may be, therefore, a need to improve medical imaging in terms of loss of time. The object of the present invention is solved by the subject-matter of the appended independent claims, wherein further embodiments are incorporated in the dependent claims, in the accompanying drawings and the following description.

According to a first aspect, there is provided a medical imaging device, comprising:
- an image acquisition unit, adapted to acquire image data of a subject to be imaged;
- a local data processing device having an artificial-intelligence-module, AI-module, adapted to automatically detect a finding on basis of the acquired image data and to determine a priority status of the detected finding; and
- a notification module, adapted to provide, if the determined priority status reaches or exceeds a notification threshold, a notification data at least containing the detected finding.

The image acquisition unit may be adapted to acquire images of a region of interest of the subject to be imaged, and may in particular adapted to use imaging technologies of X-ray radiography, magnetic resonance imaging, computer tomography, ultrasound, or the like. Accordingly, it may interact with or comprise one or more of a processing unit, a data and/or image storage etc.

The local data processing device may be a suitable computing device, comprising one or more of a processing unit, a data and/or image storage etc. It maybe arranged without a remote data connection but implemented "on system", i.e. in close spatial proximity to the image acquisition unit, e.g. within a radiologist department.

The AI-module may be implemented by program instructions utilizing machine learning techniques, in particular deep learning techniques which may be trained for that purpose, or the like. Further, it may be adapted for image classification, which may allow automatic identification of one or more findings. More specifically, the employed deep learning technique may be a convolutional neural network (CNN) consisting of one or more layers, e.g. convolutional layers, batch-normalization layers, dense layers, or the like, which may be optimized to process image data using techniques such as backpropagation. Further, according to some embodiments, the AI-module may be adapted to classify content of the image and to automatically detect the finding based on the classification. Optionally, the AI-module may be adapted to determine whether a further examination and/or treatment may be appropriate, wherein the determined additional examination and/or treatment is added to the notification data.

In this description, the term "priority status" can generally be understood as a distinction as to whether a finding is critical and therefore, e.g. has to be treated within a very short time, or whether the finding need not be prioritized. For example, the priority status may have a higher value corresponding to a critical finding and should therefore have a high priority, or may have a lower value that corresponding to a less critical finding and should therefore not have a high priority. Examples for a critical finding may comprise at least signs of a pneumothorax, an arterial dissection, or the like. Illustratively stated, a high priority status may mean a flag or special identification as a critical finding. In other words, the AI-module may be adapted for an automatic prioritization based on processing of the acquired image data.

The notification module may be implemented by program instructions, by electronic components or a combination thereof. It may be implemented within the data processing device that also has the AI-module. Alternatively, the notification module maybe implemented in a further data processing device, or the like. In some embodiments, the notification module may comprise a communication data interface that enables a data connection via a data network, a telecommunications network, e.g. a cellular or mobile network, a radio network, or the like. In general, the notification module may be adapted to transmit and/or receive text messages, combined text-picture messages etc., which may also be provided as a push message. For example, the notification contains a text-based description of the finding which may be obtained from the AI-module. The notification may be encrypted for data security.

In this description, the term "notification threshold" may be related to a preselection of critical finding, some of which may require the notification to be generated, but others may not, wherein the notification threshold makes these distinguishable. The critical finding may be included in a list, may be flagged etc.

An effect of this medical device is that a untimely communication and/or miscommunicated finding, in particular of a critical and/or time-sensitive finding, may be overcome. In more detail, an actionable finding may be detected directly during the image acquisitions process and not only after submission to a workstation or the like. Further, an automatic detection and prioritization of the finding may allow to inform a radiologist, physician etc. about the at least one potential finding in a timely manner, for example, within minutes. At the time of notification, the subject may, in the best case, not have yet left the diagnostic location, so that additional time to re-order the subject can be saved. Additional examinations, which may be advisable for the finding, can then be carried out promptly.

In an embodiment, the notification module is adapted to provide the notification data to a local display device of the imaging device.

The local display device may be, for example, a display of a system operator console which is operated and/or monitored during imaging by a technician.

Thus, at least the technician so that he can inform the radiologist about it. According to an embodiment, the notification module is adapted to provide the notification data to a first remote terminal.

The remote terminal may be a remoted but stationary device, e.g. a personal computer, or may be a portable device, e.g. a mobile phone, a tablet computer, pager etc. The remote terminal may be adapted to receive the notification data transmitted by the notification module via remote data transmission using a suitable communications protocol. Further, it may be adapted to employ different notification or messaging technologies using different communication paths, such as push-up notification, e-mail, short message service (SMS) etc.

Thus, radiologists or physicians may be notified actively about the presence of a critical finding, even if not monitoring a worklist, system operator console or the like. For example, they may be notified even if attending staff meetings, during break etc.

In an embodiment, the notification module is adapted to request an acknowledgment of receipt and/or a reading confirmation for the notification data from the first remote terminal.

The data connection may at least temporarily be bidirectional. The reading confirmation may be implemented in or provided by the messaging technology used for notification.

Thus, this may allow further subsequent actions, in particular if the notification cannot be delivered to the intended recipient. For example, the notification may be escalated to another recipient.

According to an embodiment, the notification module may be adapted, if the reading confirmation is not received within a certain time period, to notify the first remote terminal again via a second communication path that is different to a first communication path through which the reading confirmation is not received within the certain time period. The certain time period maybe dependent from e.g. the detected finding, the notification threshold, or the like. Therefore, the certain time period may be minutes, hours etc. In this description, the term "communication path" may refer to a particular communication technology, like e-mail, SMS, or the like.

Thus, it may again be attempted to notify the intended recipient before the notification is escalated and a substitute recipient is called in.

In an embodiment, the notification module is adapted, if the reading confirmation is not received within a certain time period, to provide the notification to a second remote terminal.

Thus, the notification is escalated to another recipient to lose as little time as possible to communicate the detected finding.

According to an embodiment, the notification to at least the first remote terminal is logged in a log data record.

This may primarily serve documentation purposes, but also quality assurance.

According to an embodiment, the AI-module is adapted to determine a spatial location of the finding within the subject to be imaged,
wherein the determined spatial location is added to the notification data.

The spatial location may be included text-based etc.

Thus, the radiologist or physician may be notified with a higher degree of information. This facilitates confirmation of the automatic detection performed by the AI-module.

In an embodiment, the AI-module is adapted to determine at least one image of the image data that represents at least a part and/or a partial view of the finding, and wherein the determined image is at least partly added to the notification data.

The notification may therefore be a combination of a text message and a picture message.

Thus, the radiologist or physician may be notified with a higher degree of information. This further facilitates a timely confirmation of the automatic detection performed by the AI-module.

According to an embodiment, the AI-module may be adapted to determine a likelihood of detection indicating the likelihood with which the detected finding has been correctly identified, and
wherein the determined likelihood of detection is added to the notification data.

The likelihood of detection may be determined by a machine learning algorithm or the like. For an intuitive perception, it may be indicated in percentage or the like.

Thus, the radiologist or physician may be notified with a higher degree of information. This further facilitates a timely confirmation of the automatic detection performed by the AI-module.

In an embodiment, the AI-module may be adapted to perform detecting of the finding and/or providing the notification data exclusively through local data processing. By direct data processing and eliminating other data processing instances, the notification may be provided within a particularly short time period.

According to a second aspect, there is provided a medical imaging system, comprising:
- a medical imaging device according to the first aspect; and
- a receiving device, adapted to receive notification data transmitted by the medical imaging device.

The receiving device may, for example, be the system operator console of the medical imaging device. Alternatively or additionally, if more than one receiving devices shall be notified, the receiving device may be a remote terminal, e.g. a remoted, stationary or portable device.

In an embodiment, a further remote clinical system may be connected to an image acquisition unit of the medical imaging device,
wherein the medical imaging system may be adapted to perform a pre-processing of the image by a local data processing means and providing notification data to the receiving data prior to perform a main-processing of the image data by the clinical picture archiving and communication system.

The further clinical system may be a Picture Archiving and Communication System (PACS), EMR, or the like, using communication standards like DICOM or HL-7. Using direct data processing and eliminating other data processing instances, the notification may be provided within a particularly short time period.

According to a third aspect, there is provided method of operating an medical imaging device. The method may in particular be performed using a medical imaging device according to the first aspect. The method comprises:
acquiring image data of an subject to be imaged by an imaging device,
processing the acquired image data by an imaging device-sided artificial-intelligence-module, AI-module, of a local data processing device to automatically detect a finding in the subject,
determining a priority status of the detected finding by the AI-module, and
providing a notification, containing at least the finding, to a notification module, if the determined priority status reaches or exceeds a notification threshold.

According to a fourth aspect, there is provided a computer program element for operating a medical imaging device, which, when being executed by a processing unit, is adapted to perform the method according to the third aspect.

According to a fifth aspect, there is provided a computer-readable medium having stored the computer program element according to the fourth aspect.

These and other aspects of the present invention will become apparent from and elucidated with reference to the embodiments described hereinafter.

### BRIEF DESCRIPTION OF THE DRAWINGS

Exemplary embodiments of the invention will be described in the following with reference to the following drawings:
Fig 1 shows schematically an embodiment of a medical imaging device in a side view.
Fig. 2 shows schematically a block diagram of an exemplary operation of a medical imaging device.
Fig. 3 shows schematically a block diagram of another exemplary operation of a medical imaging device.

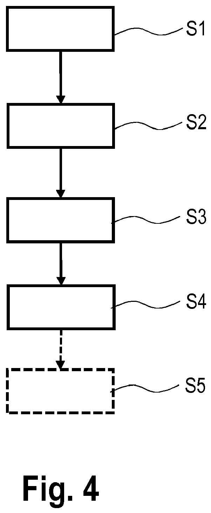

Fig. 4 shows a flow chart of a method of imaging an object by an X-ray imaging system.

The figures are merely schematic representations and serve only to illustrate embodiments of the invention. Identical or equivalent elements are in principle provided with the same reference signs.

### DETAILED DESCRIPTION OF EMBODIMENTS

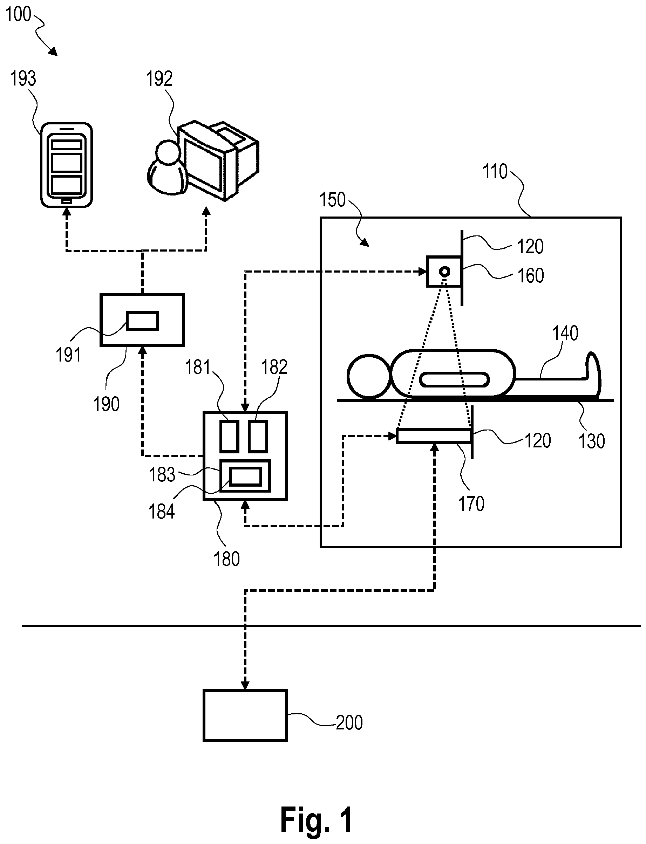

Figure 1 shows schematically a medical imaging device 100, which is in this embodiment a computed tomography imaging scanner. The X-ray imaging system 100 comprises a stationary housing 110 and a rotatable gantry 120 which is rotatable over an angular range of about 360° about a subject support 130, which is in this embodiment a support table. In this embodiment, a subject 140 to be imaged, which is exemplarily a human patient, is located on an upper surface of the subject support 130. The medical imaging device 100 further comprises an image acquisition unit 150 having a radiation source 160 that is in this embodiment configured to emit an X-ray radiation beam towards the subject 140 to be imaged, and in particular configured to generate the radiation beam to be directed into an examination region. The radiation beam interacts with a region of interest of the object 140 disposed in the examination region, wherein spatially varying absorption of the radiation is generated, as it passes through the examination region.

The medical imaging device 100 in this embodiment further comprises an X-ray detector 170 configured to detect X-rays which have passed through the subject 140 and in particular configured to detect an absorption-attenuated radiation after having passed through the examination region. In this embodiment, the radiation source 160 and the X-ray detector 170 are mounted to the gantry 120 and are arranged opposite each other, so that the X-ray detector 170 continuously receives X-rays from the radiation source 160. The X-ray detector 170 may comprise a two-dimensional array of detector elements, wherein other embodiments may be contemplated.

The medical imaging 100 further comprises one or more computational means, wherein in this embodiment mainly a local data processing device 180 will be described. In some embodiments, the data processing device 180 is connected to at least the X-ray detector 170 and/or the radiation source 160 to control these and/or to at least obtain data therefrom, in particular from the X-ray detector 170. The data processing device 180 may also be formed by several subsystems, function modules or units, software modules or units, or the like (not further detailed here), and is configured to reconstruct an image of the subject 140 based on the X-rays detected by the X-ray detector 170, and in particular based on a plurality of acquired projection images of the subject 140. In this embodiment, the data processing device 180 comprises at least one processor 181, at least one memory 182 for storing image data and at least one memory 183 for storing one or more program elements.

The data processing device 180 further comprises an artificial intelligence module, AI-module, which for better illustration is denoted by reference sign 184. The AI-module 184 has a data connection to the processor 181 and/or the X-ray detector 170 to obtain image data already processed by the processor 181 or raw image data directly provided by the X-ray detector 170. The AI-module 184 further comprises one or more artificial neural networks which may use one or more layers, e.g. convolutional layers, batch-normalization layers, dense layers, backpropagation, or the like, and which may be in particular provided as a convolutional neural network (CNN) adapted to process image data. Further, according to some embodiments, the AI-module 184 may alternatively or additionally include a deep learning algorithm and/or classification means. The CNN, classification means etc. may be pre-trained with suitable training data sets. Additionally, such a training takes place during ongoing operation to further improve the detection result. The image data is provided to the AI-module 184 as an input variable. On this basis, the AI-module 184 is adapted to automatically detect a finding on basis of the acquired image data and to determine a priority status of the detected finding. Further, the AI-module 184 is adapted to determine a spatial location of the finding within the subject 140. Also, the AI-module 184 is adapted to determine at least one image of the image data that represents at least a partial view of the finding. The AI-module 184 is further adapted to determine a likelihood of detection indicating the likelihood with which the detected finding has been correctly identified. The likelihood may be indicated in percentage or another suitable unit of measurement.

Further referring to Figure 1, the medical imaging device 100 further comprises a notification module 190 that is connected to the AI-module 184. The notification module 190 may be implemented within the data processing device 180 or may, alternatively, be implemented in a further data processing device, or the like. In this embodiment, the notification module 190 comprises a communication data interface 191, adapted to enable a data connection via a data network, a telecommunications network, e.g. a cellular or mobile network, a radio network, or the like. Further, the notification module 190 is adapted to transmit and/or receive text messages, combined text-picture messages etc., which may also be provided as a push message. The notification message transmitted by the notification module 190 maybe encrypted for data security.

As shown in Figure, 1 the notification module 190 is adapted to transmit its notification to a system operator console 192 of the medical imaging device 100, which is operated by a technician, medical support staff, or the like. Alternatively or additionally, the notification module 190 is adapted to transmit its notification to a remote terminal 193, which in this embodiment is a portable terminal, such as a mobile phone, or the like. In particular, the terminal 193 may be carried on-person by a radiologist, physician, or the like. In this embodiment, the medical imaging device 100 is connected to a further remote clinical system 200 which in this embodiment is a Picture Archiving and Communication System (PACS), EMR, or the like, using communication standards like DICOM or HL-7. It is noted that the AI-module 184 is in particular adapted to perform a pre-processing of the image data prior to providing the image data to the clinical system 200 where a subsequent main-processing of the image data is performed.

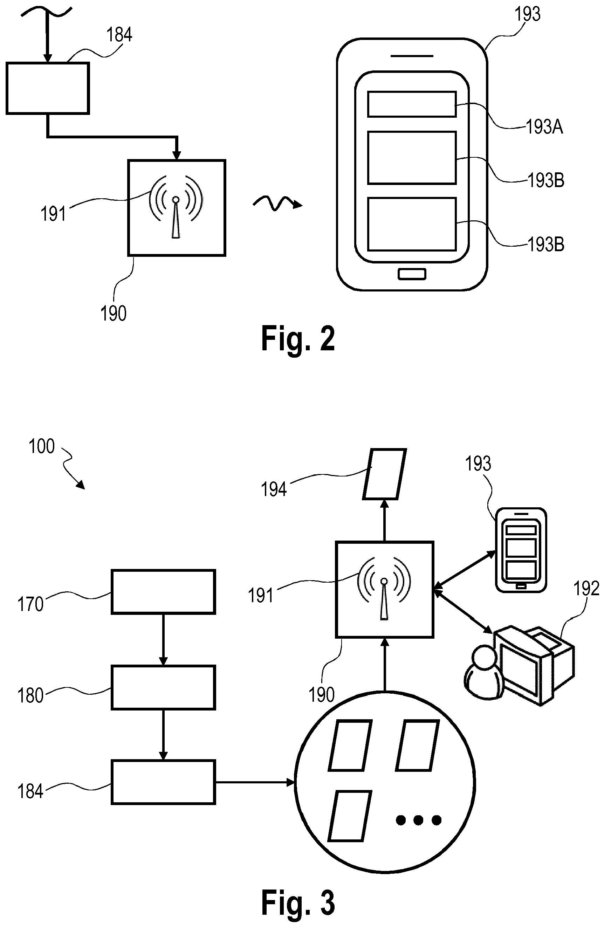

Figure 2 shows a schematic block diagram of an exemplary notification operation of the medical imaging device 100 notifying one or more of the system operator console 192 and the terminal 193 via the communication data interface 191. By way of example, the notification message of the notification module 190 contains one or more data fields, wherein in this embodiment for better illustration display fields corresponding to the data fields are denoted by reference signs 190A, 190B and 190C. In display field 190A, a notification text may be included, such as "Notification: Actionable Finding Detected for Patient ##; Pneumothorax in right lung (98%)". Accordingly, the notification may include a text indicating the detected finding, an identification data of the subject 140, and the determined likelihood. It is noted that the notification may also include additional data regarding the subject 140 such as age, gender, known clinical findings or the like. These data may be obtained by the AI-module 184, a patient information system, the clinical system 200, or the like. In display field 190B, one of the acquired images of the subject 140, in particular of the finding, may be displayed. In display field 190C, an enlarged view of an image of the finding may be displayed. For example, suitable images, especially meaningful images, to be transmitted with the notification are selected by the AI-module 184. These notification data are provided to the notification module 190 which transmits the same to the terminal 193 and/or the operator console 192.

Figure 3 shows a schematic block diagram of generating and sending the notification to be transmitted by the notification module 190. As explained above, from the X-ray detector 170 and/or the data processing device 180 acquired image data is provided to the AI-module 184, where an automatic processing of the image data is carried out with the aim of automatically detecting a possible abnormality to determine a possible finding. The AI-module 184 determines if a priority status of the finding reaches or exceeds a notification threshold, i.e. if the finding is that critical that it triggers a notification action. If the AI-module 184 determines the detected finding as critical or actionable and/or if the likelihood of detection is too low, the AI-module 184 generates several data contents for the above-mentioned notification message to be transmitted. For example, the AI-module 184 generates the naming of the finding, such as pneumothorax etc., the likelihood of detection, the spatial location of the finding etc. In some embodiments, also an appropriate pictorial representation of the finding is determined and/or generated. Then, the data contents generated by the AI-module 184 are provided to the notification module 190. In some embodiments, the notification module 190 is adapted to employ different notification or messaging technologies using different communication paths, such as push-up notification, e-mail, short message service (SMS) etc. In this embodiment, the notification is transmitted to the system operating console 192 and/or the terminal 193. In some embodiments, the notification module 190 requests an acknowledgment of receipt and/or a reading confirmation for the notification data from the remote terminal 193, as indicated in Figure 3 by the double arrow. Accordingly, the data connection between the system operating console 192 and/or the terminal 193 and the notification module 190 is at least temporarily bidirectional. The reading confirmation may be implemented in or provided by the messaging technology used for notification. If the notification cannot be delivered to the intended recipient, the notification is be escalated to another recipient, i.e. another terminal 193 (not shown). If the reading confirmation is not received by the notification module 190 within a certain time period, it notifies the terminal 193 again via a second communication path, e.g. SMS, that is different to a first communication path, e.g. e-mail, through which the reading confirmation is not received within the certain time period. The certain time period may be dependent from priority status of the detected finding, the reached or exceeded notification threshold, or the like. Therefore, the certain time period may be minutes, hours etc. As indicated in Figure 3, the notification module 190 logs the notification process in a data record 194.

Figure 6 shows a flow chart of a method of operating the medical imaging device 100 and/or medical imaging system. In a step S1, by use of e.g. the radiation source 160 and the X-ray detector 170 image data of the subject 140 is acquired.

In a step S2, the AI-module 184 processes the acquired image data to automatically detect a finding in the subject 140.

In a step S3, the AI-module 184 determines the priority status of the detected finding.

In a step S4, the AI-module 184 provides a notification, containing at least the naming of the finding, to the notification module 190, if the determined priority status reaches or exceeds a notification threshold.

In an optional step S5, the notification module 190 transmits the notification as a notification message to one or more of the system operator console 192 and the remote terminal 193.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program maybe stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.
However, the computer program may also be presented over a network like the internet and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: medical imaging device
- 110: housing
- 120: gantry
- 130: subject support
- 140: subject to be imaged
- 150: image acquisition unit
- 160: radiation source
- 170: X-ray detector
- 180: data processing means
- 181: processor
- 182: memory
- 183: memory
- 184: artificial-intelligence-module
- 190: notification module
- 191: communication data face
- 192: system operator console
- 193: remote terminal
- 194: data record
- 200: further clinical system

## Claims

1. A medical imaging device (100), comprising:
an image acquisition unit (150), adapted to acquire image data of an subject (140) to be imaged,
a local data processing device (180) having an artificial-intelligence-module (184), AI-module, adapted to automatically detect a finding on basis of the acquired image data and to determine a priority status of the detected finding, and
a notification module (190), adapted to provide, if the determined priority status reaches or exceeds a notification threshold, a notification data containing the detected finding.

2. The medical imaging device (100) according to claim 1,
wherein the notification module (190) is adapted to provide the notification data to a local display device of the imaging device.

3. The medical imaging device (100) according to claim 1 or 2,
wherein the notification module (190) is adapted to provide the notification data to a first remote terminal (193).

4. The medical imaging device (100) according to claim 3,
wherein the notification module (190) is adapted to request a reading confirmation for the notification data from the first remote terminal (193).

5. The medical imaging device (100) according to claim 4,
wherein the notification module (190) is adapted, if the reading confirmation is not received within a certain time period, to notify the first remote terminal (193) again via a second communication path that is different to a first communication path through which the reading confirmation is not received within the certain time period.

6. The medical imaging device (100) according to claim 4 or 5,
wherein the notification module (190) is adapted, if the reading confirmation is not received within a certain time period, to provide the notification to a second remote terminal (193).

7. The medical imaging device (100) according to any one of the preceding claims,
wherein the AI-module (184) is adapted to determine a spatial location of the finding within the subject to be imaged, and
wherein the determined spatial location is added to the notification data.

8. The medical imaging device (100) according to any one of the preceding claims,
wherein the AI-module (184) is adapted to determine at least one image of the image data that represents at least a partial view of the finding, and
wherein the determined image is at least partly added to the notification data.

9. The medical imaging device (100) according to any one of the preceding claims,
wherein the AI-module (184) is adapted to determine a likelihood of detection indicating the likelihood with which the detected finding has been correctly identified, and
wherein the determined likelihood of detection is added to the notification data.

10. The medical imaging device (100) according to any one of the preceding claims,
wherein the AI-module (184) is adapted to perform detecting of the finding and/or providing the notification data exclusively through local data processing.

11. A medical imaging system, comprising:
a medical imaging device (100) according to any one of the preceding claims and
a receiving device (192, 193), adapted to receive notification data transmitted by the medical imaging device (100).

12. The medical imaging system according to claim 11, further comprising:
a further remote clinical system (200), connected to an image acquisition unit (150) of the medical imaging device,
wherein the medical imaging system is adapted to perform a pre-processing of the image data by a local data processing means using the medical imaging device and providing notification data to the receiving device prior to performing a main processing of the image data by the clinical system (200).

13. A method of operating an medical imaging device (100), comprising:
acquiring image data of an subject (140) to be imaged,
processing the acquired image data by an artificial-intelligence-module (184), AI-module, of a local data processing device (180) to automatically detect a finding in the subject (140),
determining a priority status of the detected finding by the AI-module (184), and
providing a notification, containing at least the finding, to a notification module (190), if the determined priority status reaches or exceeds a notification threshold.

14. A computer program element for operating a medical imaging device (100), which, when being executed by a processing unit, is adapted to perform the method according to claim 13.

15. A computer-readable medium having stored the computer program element of claim 14.
